# EUROPEAN PATENT SPECIFICATION

(11) **EP 1 864 768 B1**
(45) Date of publication and mention of the grant of the patent: **11.11.2009**
(21) Application number: 07109033.6
(22) Date of filing: 28.05.2007
(51) Int. Cl.: B26D 5/20, B26D 7/18, A61F 13/15, B32B 37/00, B26D 1/62, B26D 1/40

(54) **Cutting device, for instance for producing sanitary products, and corresponding methods of operation**
Schneidvorrichtung zur Herstellung von Hygieneprodukten und entsprechende Verwendung einer solchen Vorrichtung
Dispositif de coupe pour la fabrication de produits sanitaires et procédé d'utilisation correspondant

(30) Priority: 05.06.2006 IT TO20060408
(43) Date of publication of application: 12.12.2007
(73) Proprietor: Fameccanica.Data S.p.A., 66020 Sambuceto di S. Giovanni Teatino (Chieti) (IT)
(72) Inventor: Lombardi, Massimiliano, 65016 Montesilvano (Pescara) (IT); Lupinetti, Serafino, 65010 Elice (Pescara) (IT); Giansante, Antonio, 65010 Spoltore (Pescara) (IT)
(74) Representative: Bosotti, Luciano

(56) References cited:
- EP-A- 1 069 061
- EP-A2- 0 692 375
- DE-A1- 3 444 331
- US-A- 3 963 557
- US-A- 5 380 381
- US-A- 5 407 513

## Description

The present invention relates to techniques that enable formation of laminar elements of predetermined length via cutting of a laminar material to length. The invention has been developed with particular attention paid to its possible use in the field of plants for the production of sanitary products, such as, for example, panty liners, diapers, etc.

According to one of the most widespread solutions existing today, these products are produced continuously or "in line", i.e., starting from a plurality of webs variously superimposed on and coupled to one another so as to produce a finished web, from which the individual finished articles are then obtained (usually via cutting operations).

In various steps of the production process - and also in the steps subsequent to obtaining the individual products - there can arise the need to apply, on a moving element (whether this be of a continuous type or else part of a continuous or substantially continuous flow of distinct elements), laminar elements, which, in view of the specific application, must be cut to length. Usually, the cutting operation must be performed in a cadenced way, in so far as it is designed to generate elements that may be applied with a predetermined cadence (and, more often than not, with a "phasing") with respect to the flow of the products leaving the process.

The meeting of the above needs is rendered even more critical by the fact that, more often than not, the laminar elements in question have a length that is different from (usually shorter than) the length of the articles on which they are applied.

For example, the laminar element that is to be cut to length may be the core or absorbent pad of a sanitary product having an overall length greater than the homologous length of the absorbent pad: for instance, just to give an idea, it is sufficient to consider the distance that separates the two waistbands of normal hourglass-shaped diapers, a distance that is usually greater than the antero-posterior length of the absorbent pad. Another example of laminar element that is to be cut to length and to be applied in a cadenced way on a flow of articles is constituted by the label or labels (usually siliconized paper) that are to be applied on the areas of a sanitary article treated with adhesive material. As typical example, reference may be made to sanitary pads for ladies of the type commonly referred to as "panty liners with wings". In the finished absorbent article, three adhesivized areas are present, one extending along the principal median axis of the pantie liner, to enable exact positioning thereof on the crotch portion of the panties of the user, and another two, of smaller length, located in the area corresponding to the wings to enable adhesive application of the wings themselves in the folded condition underneath the crotch portion of the panties. In all three cases, the non-adhesive label has a length smaller (in the case of the labels that are to be applied on the wings, much smaller) than the overall length of the product. There exists then the need to get the label: (i) to be cut to the desired length, and (ii) to be applied precisely in the position corresponding to the adhesivized area that the label must protect.

To meet the above needs, machines are currently used based upon a configuration that can be defined as transfer configuration; the material constituting the labels, fed in the form of a web, is first subjected to a cutting operation via a cutting head provided with rotary blades acting on a reception counter-blade, which also sees to the subsequent application of the labels on the advancing flow of articles.

A substantially similar solution is known from US-A-3 963 557, where the function of the aforesaid counter-blade is performed by rotary-applicator segments.

The plants currently used in the sector must enable production of different products and/or the same product in different formats. With the consequent need to have available elements cut to different lengths, it is necessary to use cutting and/or application devices of a different type, which must be each time alternated in use to obtain the products with the desired characteristics. All this results in an increase in the global cost of the plant and, above all, in the need to stop the plant, even for quite prolonged time intervals, when passing to the production of an article with different characteristics with respect to the article treated previously (the so-called "format change").

US-B-6 269 720 describes an improved solution, in which the laminar elements, constituted, for example, by labels to be applied on moving articles, are formed starting from a web coming from a supply unit. The web is fed to a reception unit passing through a cutting area. The individual labels are formed, controlling intervention of the cutting unit when the amount of web supplied by the supply unit corresponds to the desired length of the labels. The cutting unit preferentially comprises a rotary blade and a counter-blade, constituted by a non-motor-driven roller supported in a lubricated cradle and loaded elastically against a cutting unit. Preferentially, the reception unit provides also for application of the elements cut to length on the respective articles. The solution known from US-B-6 269 720 thus frees the element that is traditionally to co-operate with the blade or the blades in the cutting operation (the so-called counter-blade) from the need to perform also the cadenced transfer of the elements obtained via the cutting operation. In particular, the solution described in US-B-6 269 720 envisages the use, as counter-blade, of a roller of elongated and slim shape, with minimal diametral dimensions and not motor-driven; immediately located downstream of the counter-blade is a transfer belt actuated starting from the main motor drive of the plant for transfer of the articles: it is thus possible to adapt the speed automatically to the rate of flow of the articles. Preferentially, the aforesaid counter-blade is mounted on a particular lubricated cradle, subjected to elastic load, that is able to distribute the shearing stress adequately.

More specifically, the invention relates to a method of actuating a cutting device according to the preamble of claim 1, which device is known e.g. from DE 3 444 331 A1. Substantially similar devices are known e.g. US-A-5 380 381, US-A-5 407 513, US-A-3 963 557, EP-A-1 069 061 or EP-A2-0 692 375.

Even though the above solutions according to the prior art have proven altogether functional from the operating standpoint, they may undergo further improvements from different points of view, such as, for example:
- the general simplification of the device, in particular as regards minimization of the component parts; and
- the possibility of operating in conceptually "pitchless" conditions, i.e., with the device that applies the material that has previously been cut at a rate equal or very close to that of the web of the main process.

The above improvements enable the consequent possibility of:
- avoiding the replacement/modification of the device for change of size or change of format;
- avoiding the use of hard-metal (HM) inserts in the cutting unit; and
- increasing the useful working life of the device.

The object of the present invention is to provide a cutting device that is able to implement such an improvement.

According to the present invention, the above object is achieved thanks to a method having the characteristics specifically recalled in Claim 1. that follows. The claims form an integral part of the disclosure of the invention provided herein.

It is, on the other hand, evident that the field of application of the invention is not limited to the one for which the invention itself has been specifically developed. The invention is suited in fact to being applied, in general, in all those contexts in which there exists the need to perform a cutting to length/application of laminar elements in view of their possible application on a web and/or a continuous or substantially continuous flow of moving articles.

The invention will now be described, purely by way of non-limiting example, with reference to the annexed plate of drawings, which consist of a single figure constituting a general view in side elevation of a device according to the invention.

In the figure, the reference number 1 designates as a whole a device usable for the cutting to length and subsequent application of laminar elements, for example, in the framework of a plant for the production of sanitary products.

For example, the products in question can be constituted by absorbent sanitary articles that advance in a continuous flow (for instance, because they are still connected to one another in the form of a web W) on a conveyor (not illustrated, but of a known type, for example, of the motor-driven-belt type), located in a position underlying the device 1.

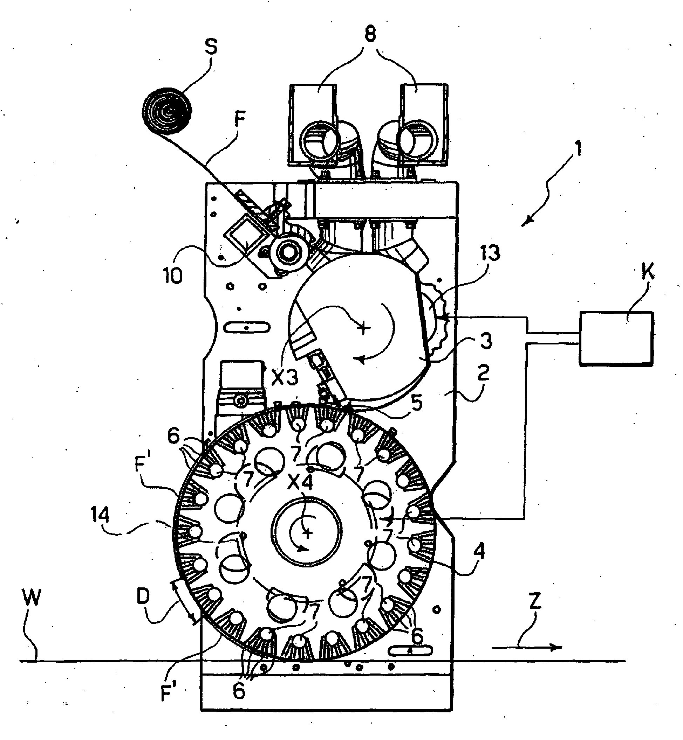

With reference to the viewpoint of Figure 1, it is assumed that the web of articles W moves from left to right (direction designated by z).

The deliberately schematic character of this representation explains very well the fact that the range of possible application of the solution described herein is altogether general. This applies also as regards the nature and the characteristics of a sheet material F, which, rolling off a source S, is to be segmented into lengths F', which are in turn to be applied on the web W in a synchronous and regular way, with the lengths F' separated by a distance D. The above process is performed, ensuring at the same time that the action of application of the lengths F' is "phased" with the advance of the web W: this, for example, to ensure that each length F' is applied on a respective article or product of the web W in an exactly determined position (for example, at half the length) of the article or product.

Just to give an idea (without this implying any limitation to the scope of the invention), it is possible to consider the device 1 illustrated herein as performing the operation of cutting and application of a non-adherent label F' of siliconized paper along the principal longitudinal axis of each absorbent article A. In the case where the latter is an article of the type with wings, an ensemble of parts substantially similar to what has been described performs the operations of cutting and application of adhesive labels, which are to be applied on the wings of the absorbent article.

The device 1 is made up basically of a sturdy supporting structure 2, for example made of metal, which supports, in cantilever fashion, two counterrotating rollers 3 and 4, which turn about respective axes X3 and X4 (horizontal, in the example of use illustrated here).

The roller 3 (the term "roller" refers basically to its character of rolling body, and is in no way tied down to the choice of an exactly cylindrical shape) is mounted in a top position and functions basically as rotary blade that is to act on the material F to section it into successive lengths F'.

The roller 4, mounted in a bottom position, functions, instead, as counter-blade (or "counter-roller": the two terms can in effect be considered equivalent to one another).

The sheet material F arriving from the source S advances through a reception and guide structure 10 (such as an unwinder with motor-driven rollers); from here it is wound on around the roller 3 and then passes into the space or gap between the two rollers 3 and 4, where the material F will be segmented into lengths F', as described in greater detail in what follows. Finally, the material F (or, rather, the lengths F' separated by a distance D) is "accompanied" by the roller 4 that applies it to the web W.

Once applied on the web W, the lengths F' are withheld there with known means (for example, adhesively); the corresponding details, in themselves known, do not assume any specific importance for the purposes of the solution described herein.

The rollers 3 and 4 are moved in opposite directions and independently of one another by respective motor drives 13, 14 (only partially visible in the figure, but of a known type), which causes them to rotate, respectively, in a clockwise direction (roller 3) and in a counterclockwise direction (roller 4).

The roller 3 functioning as blade carries a cutting edge 5 that orbits about the axis X3, performing a number of turns, dictated in principle by the number of pieces per minute treated by the machine (i.e., to provide a simple example, by the number of non-adhesive labels applied on a corresponding number of absorbent sanitary articles per unit time).

In actual fact, we are considering the (average) number of turns given that, as will be explained in greater detail in what follows, the motor drive of the roller 3 is able to impart upon the cutting edge (or blade proper) 5 an orbital speed about the axis X3 that varies according to a "virtual" or "electronic" cam law.

The roller 4, which functions as counter-blade, is perforated in a radial direction with holes provided in its skirt surface and ordered in rows 6 extending along the generatrices of the roller 4, hence in directions parallel and concentric with respect to the axis X4 of the roller 4 itself.

The holes of the rows 6 come under respective manifolds 7, which in turn are connected to a line for supplying subatmospheric pressure (the so-called "vacuum" line). Two intake mouths of this line, designated by 8, are visible in the upper part of the figure.

According to criteria that are well known from similar applications, when the "vacuum" line is activated, through the holes 6 a differential of pressure/flow of intake air is set up, the effect of which is to withhold the sheet material F and the lengths F' cut therefrom adherent to the counter-blade 4, thus enabling drawing-along thereof by the counter-blade in non-rigid conditions, in the sense that the sheet material F is possibly able to slide tangentially on the skirt surface of the counter-blade 4.

The above "vacuum" action of retention enables the counter-blade 4 to bring the elements F' towards the web W, obtaining application thereof in the desired position. Usually this occurs by causing the vacuum action of retention of the elements F' by the counter-blade 4 to cease in the area of application on the web W: also here the corresponding details, which are in themselves known, do not assume any specific importance for the purposes of the solution described herein.

The rows 6 of holes distributed along the perimeter of the skirt of the counter-blade 4 identify as many positions of possible interaction with the cutting edge 5, i.e., positions in which, by interacting with one another with the interposition of the material F, the cutting edge 5 and the skirt surface of the counter-blade 4 determine cutting to length of the material F and consequent formation of the lengths or elements F'. The aforesaid positions of interaction are chosen substantially (usually, exactly) at equal distances apart from one another, and are set at a distance sufficiently small to form in effect a sort of *continuum* of possible positions of interaction.

The number of rows 6 of holes (i.e., the number of positions of interaction with the cutting edge 5) distributed along the perimeter of the skirt of the counter-blade 4 is a prime number n, for example 83: this number - with a value given purely by way of example - refers to a device 1 of normal dimensions, with the rollers 3 and 4 having diameters in the region of 170 and 320 mm, respectively.

The choice of the specific value of the prime number n arises as compromise dictated by the need to proceed so that the circumferential pitch between the rows 6 of holes is sufficiently small, for example 10-12 mm.

The speed of rotation of the counter-blade roller 4 is in fact chosen in such a way that the peripheral speed (or tangential speed) of the counter-blade 4 itself is substantially - but not exactly - equal to the linear speed of the web W that moves tangentially with respect to the lowest region of the counter-blade 4.

In particular, assuming that the length of the individual product treated comprised in the web W corresponds to a certain (non-integer) number of pitches between the rows 6 of holes of the counter-blade 4, the counter-blade 4 is made to rotate with a motion such that, in the interval necessary for "scanning" the length of the product in question, the skirt surface of the counter-blade 4 advances in actual fact by a distance given by a number of pitches between the rows 6 equal to the immediately adjacent integer number that approximates by defect the aforesaid non-integer number. It will be appreciated, on the other hand, that, even though the corresponding solutions do not appear of effective practical interest, the choice could also fall - at least in principle - on an integer number that approximates by excess the aforesaid non-integer number and/or on a number from amongst the integer numbers not immediately adjacent to the aforesaid non-integer number.

For example, suppose we have:
- length of product: 550 mm
- distance or pitch between the rows of holes 6: 12.11 mm
- number of pitches necessary for covering 550 mm: 550/12.11 = 45.41
- advance imparted on the counter-blade in number of pitches: 45.

In the example of embodiment considered here, the total number of the rows 6 of holes is, as has been seen, 83. An advance of 45 pitches per product thus corresponds to 45/83 = 0.54 turns of the counter-blade for each individual product.

The above procedure (and in particular the fact that the number n of rows 6 is a prime number) ensures that, whenever the blade 3 acts with its cutting edge 5 on the material F, this occurs in sequence in the area corresponding to a different row of holes 6. The corresponding stress is thus distributed over time in the sense that each row 6 of holes is involved only once out of n times in the cutting operation. In other words, of n successive cutting operations, only one involves a given row 6 of holes.

Of course, even though for reasons of simplicity and clarity of illustration, reference has been made to cutting operations that involve each time a row of holes 6, in actual fact the movement of the blade 3 and of the counter-blade 4 is adjusted so as to cause the cutting edge 5 to "hit" on the counter-blade 4 in the space between two adjacent rows of holes 6, i.e., on a continuous part of the skirt surface of the counter-blade 4.

To ensure proper operation of the device 1, the peripheral speed of the counter-blade 4 is chosen equal to or higher than the peripheral speed of the blade 3, with a maximum deviation of the speed between the two rollers 3 and 4 such as to prevent excessive values of "relative sliding", for example values not higher than approximately 130 mm.

It is, on the other hand, possible to envisage also larger deviations by resorting to an "electronic cam" provided by the servo motor for driving the blade 4 that enables the unit to process a very wide range of lengths, but limiting (or even eliminating) the relative sliding between blade and counter-blade at the instant of cutting.

For a better understanding of this concept, with reference to the ensuing table, three formats (lengths) of product (small, average, and large) may be considered:

| Format | Theoretical develop. (mm) | Turns of blade | Pitches of counter-blade | Turns of counter-blade | Sliding between blade and counter-blade (mm) |
|---|---|---|---|---|---|
| Small | 558.3 | 1 | 46 | 0.55 | 134 |
| Average | 690.1 | 1 | 57 | 0.69 | 0.88 |
| Large | 791.7 | 1 | 65 | 0.78 | -96 |

The diameter of the blade 3 can hence be chosen, for example, equal to 691.15 mm dev/turn/prod, (where the expression "dev/turn/prod" means that in one complete rotation of the blade the linear measurement of the circumference, expressed in mm, is linked to the passage on the machine of a complete product understood as length or pitch, that is, referred to the average product) in such a way as not to have practically any sliding between the two rollers 3 and 4 in the instant of cutting for this size of products.

In the case of the large product, the counter-blade 4 for one product performs approximately +94 mm of sliding with respect to the blade, and the application is in this case correct (thanks to the possibility of sliding of the material F with respect to the surface of the counter-blade 4). What, instead, is not correct is the case of the small size because the counter-blade 4 for one product performs approximately -134 mm of sliding with respect to the blade 3: in this case (by performing the function of "electronic cam") the servo motor that drives the blade 3 intervenes by decelerating the blade 3 in the instant of cutting, also in this case bringing back to 0 mm the sliding between the axes at the moment of cutting.

In the remaining path that the blade 3 has to perform to make a complete revolution, the servo motor accelerates the blade 3 itself to reposition it in conditions such as to be able to repeat the cycle continuously. The cycle is completed in the passage of one product through the main process.

The diameter of the counter-blade 4, in addition to being influenced by the relation existing between the pitch between the rows 6 of holes (of approximately 10-12 mm) and the (prime) number n of said rows, is also preferably linked to the length of the piece F' that is to be cut and subsequently applied to the web W.

Of course, without prejudice to the principle of the invention, the details of construction and the embodiments may vary widely with respect to what is described and illustrated herein, without thereby departing from the scope of the invention, as defined in the claims that follow.

## Claims

1. A method of actuating a device for forming laminar elements (F') of predetermined length by cutting to length a laminar material (F), the device comprising:
- a first rotary element (3), to receive wound thereon said laminar material (F) and carrying at least one orbiting cutting edge (5), to act on said laminar material; and
- a second rotary element (4) having a skirt surface defining with respect to said first rotary element (3) a gap of passage for said laminar material (F); said skirt surface to interact with said cutting edge (5) with the interposition of said laminar material (F) to carry out cutting to length of the laminar material (F) itself and thus forming said laminar elements (F'),
in which said first rotary element (3) and second rotary element (4) are controllable in rotation in opposite directions independently of one another and said skirt surface of said second rotary element (4) has a given number of positions of interaction with said cutting edge (5) carried by said first rotary element (3), said positions substantially being set at equal distances apart,
**characterized in that** the method includes the operations of:
- making laminar elements (F') to be applied via said second rotary element (4) on a flow of moving products (W) with a application pitch corresponding to a non-integer multiple of the distance of separation between said positions of interaction with said cutting edge (5), and
- controlling the rotation of said second rotary element (4) by causing said skirt surface to advance with a speed that is substantially, but not exactly, the same as the speed of said flow of moving products (W), so that, for each application pitch, said skirt surface advances by an integer number of said positions of interaction that approximates said non-integer multiple.

2. The method according to Claim 1, **characterized in that**, for each application pitch, said skirt surface is made to advance by an integer number of said positions of interaction that approximates by defect said non-integer multiple.

3. The method according to Claim 1 or Claim 2, **characterized in that**, for each application pitch, said skirt surface is made to advance by an integer number of said positions of interaction chosen from among the integer numbers immediately adjacent to said non-integer multiple.

4. The method according to Claim 1, **characterized in that** said given number of positions of interaction is a prime number.

5. The method according to Claim 1, **characterized in that** said skirt surface of said second rotary element (4) is provided with openings (6) connectable (7, 8) to a source of subatmospheric pressure for withholding said laminar material (F) and said laminar elements (F') on said skirt surface.

6. The method according to Claim 5, **characterized in that** said openings (6) are ordered in rows extending along the generatrices of said skirt surface and distributed on the contour of said skirt surface.

7. The method according to any one of Claim 1 or Claim 4, taken in combination with Claim 6, **characterized in that** said rows of openings (6) identify said positions of interaction with said cutting edge (5) carried by said first rotary element (3).

8. The method according to any one of Claims 1 to 7, **characterized in that** it comprises the operation of controlling the rotation of said first (3) and second (4) rotary elements in mutually opposite directions and determining selectively, when said skirt surface interacts with said cutting edge (5) with the interposition of said laminar material (F), a relative sliding in the sense of a slowing-down of said cutting edge (5) with respect to said skirt surface of said second rotary element (4).

9. The method according to Claim 8, **characterized in that** it comprises the operation of accelerating the movement of rotation of said first rotary element (3) after said skirt surface has interacted with said cutting edge (5) with the interposition of said laminar material (F).

10. The method according to Claim 8 or Claim 9, **characterized in that** it comprises the operation of getting said first rotary element (3) to perform a complete turn for each interaction with said skirt surface.

## Patentansprüche

1. Verfahren zum Bedienen einer Vorrichtung zum Bilden laminarer Elemente (F') von vorbestimmter Länge durch Auf-Länge-Schneiden eines laminaren Materials (F), wobei die Vorrichtung umfasst:
- ein erstes Drehelement (3) um das laminare Material (F) darauf gewickelt aufzunehmen und zumindest eine umlaufende Schneidekante (5) aufzunehmen, um auf das laminare Material zu wirken; und
- ein zweites Drehelement (4), welches eine Randleistenoberfläche aufweist, die in Bezug auf das erste Drehelement (3) einen Durchgangszwischenraum für das erste laminare Material (F) definiert, wobei die Randleistenoberfläche mit der Schneidekante (5) mit der Zwischenlagerung des laminaren Materials (F) selbst zusammenwirken soll, um das Auf-Länge-Schneiden des laminaren Materials (F) selbst auszuführen und daher die laminaren Elemente (F') bilden,
- in welchen das erste Drehelement (3) und zweite Drehelement (4) unabhängig voneinander in entgegengesetzten Richtungen drehsteuerbar sind und die Randleistenoberfläche des zweiten Drehelements (4) eine gegebene Anzahl von Positionen des Zusammenwirkens mit der Schneidekante (5) aufweist, die von dem ersten Drehelement (3) getragen wird, wobei die Position im Wesentlichen in gleichen Abständen beabstandet werden,
**dadurch gekennzeichnet, dass** das Verfahren die folgenden Arbeitsschritte einschließt:
- Herstellen laminarer Elemente (F'), um über das zweite Drehelement (4) auf einen Fluss von sich bewegenden Produkten (W) mit einem Anbringungsabstand entsprechend einem nicht ganzzahligen Vielfachen des Trennungsabstands zwischen der Interaktionsposition mit der Schneidekante (5) angebracht zu werden, und
- Kontrollieren der Drehung des zweiten Drehelements (4) durch Voranschreiten der Randleistenoberfläche mit einer Geschwindigkeit, welche im Wesentlichen, aber nicht genau dieselbe ist wie die Geschwindigkeit des Flusses sich bewegender Produkte (W) so dass die Randleistenoberfläche für jeden Anbringungsabstand um eine ganze Zahl der Zusammenwirkpositionen voranschreitet, welche das nicht ganzzahlige Vielfache annähern.

2. Verfahren nach Anspruch 1, **dadurch gekennzeichnet, dass** die Randleistenoberfläche für jeden Anbringungsabstand um eine ganze Zahl der Zusammenwirkpositionen voranschreitet, welche im Regelfall das nicht ganzzahlige Vielfache annähern.

3. Verfahren nach Anspruch 1 oder Anspruch 2, **dadurch gekennzeichnet, dass** die Randleistenoberfläche für jeden Anbringungsabstand um eine ganze Zahl der Zusammenwirkpositionen voranschreitet, welche aus den ganzen Zahlen gewählt wird, die unmittelbar an das nicht ganzzahlige Vielfache angrenzen.

4. Verfahren nach Anspruch 1, **dadurch gekennzeichnet, dass** die gegebene Anzahl von Zusammenwirkpositionen eine Primzahl ist.

5. Verfahren nach Anspruch 1, **dadurch gekennzeichnet, dass** die Randleistenfläche des zweiten Drehelements (4) mit Öffnungen (6) bereitgestellt wird, die mit einer Quelle von subatmosphärischem Druck zum Zurückhalten des laminaren Materials (F) und des laminaren Elements (F') auf der Randleistenfläche verbindbar (7, 8) sind.

6. Verfahren nach Anspruch 5, **dadurch gekennzeichnet, dass** die Öffnungen (6) in Reihen angeordnet sind, welche sich entlang der Generatricen der Randleistenfläche erstreckt und auf dem Umfang der Randleistenoberfläche verteilt ist.

7. Verfahren nach einem der Ansprüche 1 bis 4 in Kombination mit Anspruch 6, **dadurch gekennzeichnet, dass** die Öffnungsreihen (6) die Zusammenwirkpositionen mit der Schneidkante (5) identifizieren, welche von dem ersten Drehelement (3) getragen wird.

8. Verfahren nach einem der Ansprüche 1 bis 7, **dadurch gekennzeichnet, dass** es den Arbeitsschritt des Steuerns der Drehung des ersten (3) und zweiten (4) Drehelements in gegenseitig entgegengesetzte Richtungen steuert und des selektiven Bestimmens, wann die Randleistenoberfläche mit der Schneidkante (5) mit der Zwischenlagerung des laminaren Materials (F) zusammenwirkt, ein relatives Gleiten im Sinne einer Verlangsamung der Schneidkante (5) in Bezug auf die Randleistenoberfläche des zweiten Drehelements (4) umfasst.

9. Verfahren nach Anspruch 8, **dadurch gekennzeichnet, dass** es den Arbeitsschritt des Beschleunigens der Drehbewegung des ersten Drehelements (3) umfasst, nachdem die zweite Randleistenoberfläche mit der Schneidkante (5) mit der Zwischenlagerung des laminaren Materials (F) zusammengewirkt hat.

10. Verfahren nach Anspruch 8 oder 9, **dadurch gekennzeichnet, dass** es den Arbeitsschritt umfasst, das erste Drehelement (3) dazu zu bringen, eine vollständige Drehung für jedes Zusammenwirken mit der Randleistenoberfläche auszuführen.

## Revendications

1. Un procédé d'actionnement d'un dispositif pour former des éléments laminaires (F') de longueur prédéterminée par découpe à la longueur d'un matériau laminaire (F), le dispositif comprenant :
- un premier élément rotatif (3), pour recevoir enroulé dessus ledit matériau laminaire (F) et portant au moins un bord de coupe orbital (5), pour agir sur ledit matériau laminaire, et
- un second élément rotatif (4) ayant une surface de jupe définissant par rapport audit premier élément rotatif (3) un intervalle de passage pour ledit matériau laminaire (F) ; ladite surface de jupe pour interagir avec ledit bord de coupe (5) avec interposition dudit matériau laminaire (F) pour effectuer la découpe à la longueur du matériau laminaire (F) lui-même et former ainsi lesdits éléments laminaires (F'),
dans lequel ledit premier élément rotatif (3) et ledit second élément rotatif (4) peuvent être contrôlés en rotation dans des directions opposées indépendamment l'un de l'autre et ladite surface de jupe dudit second élément rotatif (4) possède un nombre donné de positions d'interaction avec ledit bord de coupe (5) porté par ledit premier élément rotatif (3), lesdites positions étant substantiellement disposées à égale distance d'éloignement l'une de l'autre,
**caractérisé en ce que** le procédé comprend les étapes suivantes :
- faire en sorte que les éléments laminaires (F') soient appliqués via ledit second élément rotatif (4) sur un flux de produit en déplacement (W) avec un pas d'application correspondant à un multiple non entier de la distance de séparation entre lesdites positions d'interaction avec ledit bord de coupe (5), et
- contrôler la rotation dudit second élément rotatif (4) en faisant en sorte que ladite surface de jupe avance avec une vitesse qui soit substantiellement, mais non exactement, la même que la vitesse dudit flux de produit en déplacement (W) de sorte que, pour chaque pas d'application, ladite surface de jupe avance d'un nombre entier desdites positions d'interaction qui correspond approximativement audit multiple non entier.

2. Le procédé selon la revendication 1, **caractérisé en ce que**, pour chaque pas d'application, on fait avancer ladite surface de jupe d'un nombre entier desdites positions d'interaction qui correspond approximativement par défaut audit multiple non entier.

3. Le procédé selon la revendication 1 ou la revendication 2, **caractérisé en ce que,** pour chaque pas d'application, on fait avancer ladite surface de jupe d'un nombre entier de positions d'interaction choisi parmi les nombres entiers immédiatement adjacents audit multiple non entier.

4. Le procédé selon la revendication 1, **caractérisé en ce que** ledit nombre donné de positions d'interaction est un nombre premier.

5. Le procédé selon la revendication 1, **caractérisé en ce que** ladite surface de jupe dudit second élément rotatif (4) est pourvue d'ouvertures (6) qui peuvent être reliées (7, 8) à une source de pression sub-atmosphérique pour retenir ledit matériau laminaire (F) et lesdits éléments laminaires (F') sur ladite surface de jupe.

6. Le procédé selon la revendication 5, **caractérisé en ce que** lesdites ouvertures (6) sont ordonnées en rangées s'étendant le long des génératrices de ladite surface de jupe et distribuées sur le contour de ladite surface de jupe.

7. Le procédé selon l'une de la revendication 1 ou de la revendication 4, prise en combinaison avec la revendication 6, **caractérisé en ce que** lesdites rangées d'ouvertures (6) identifient lesdites positions d'interaction avec ledit bord de coupe (5) porté par ledit premier élément rotatif (3).

8. Le procédé selon l'une des revendications 1 à 7, **caractérisé en ce qu'**il comprend l'opération de contrôle de la rotation desdits premier (3) et second (4) éléments rotatifs dans des directions mutuellement opposées et de détermination sélective, lorsque ladite surface de jupe interagit avec ledit bord de coupe (5) avec interposition dudit matériau laminaire (F), d'un coulissement relatif dans le sens d'un ralentissement dudit bord de coupe (5) par rapport à ladite surface de jupe dudit second élément rotatif (4).

9. Le procédé selon la revendication 8, **caractérisé en ce qu'**il comprend l'opération d'accélération du mouvement de rotation dudit premier élément rotatif (3) après que ladite surface de jupe ait interagi avec ledit bord de coupe (5) avec interposition dudit élément laminaire (F).

10. Le procédé selon la revendication 8 ou la revendication 9, **caractérisé en ce qu'**il comprend l'opération consistant à faire en sorte que le premier élément rotatif (3) exécute un tour complet pour chaque interaction avec ladite surface de jupe.
